# EUROPEAN PATENT APPLICATION

(11) **EP 1 206 911 A1**
(43) Date of publication of application: **22.05.2002**
(21) Application number: 00954917.1
(22) Date of filing: 23.08.2000
(51) Int. Cl.: A23K 1/16, A61K 39/39, A61K 39/395, A61P 43/00, A61K 38/17

(54) **GROWTH PROMOTING COMPOSITIONS FOR MAMMALS AND METHOD OF PROMOTING THE GROWTH OF MAMMALS BY USING THE SAME**

(30) Priority: 24.08.1999 JP 23628799
(71) Applicant: Meiji Seika Kaisha, Ltd., Tokyo 104-8002 (JP)
(72) Inventor: OTANI, Hajime, Faculty Agriculture Shinshu Univ., Kamiina-gun, Nagano 399-4511 (JP); KITAMURA, Hiroshi, Meiji Seika Kaisha Ltd., Tokyo 104-8002 (JP); SAWADA, Kazuhiko, Meiji Seika Kaisha Ltd., Tokyo 104-8002 (JP); OSHIDA, Toshio, School of Veterinary Medicine, Sagamihara-shi, Kanagawa 229-0006 (JP); KUSUHARA, Seiji, Fact. Agriculture Niigata Univ., Niigata 950-2102 (JP)
(74) Representative: Gille Hrabal Struck Neidlein Prop Roos
(86) International application number: JP0005631
(87) International publication number: WO0113739

(57) **Abstract**

The invention aims to provide a method for enhancing an immune of mammals and promoting growth thereof without problems in safety, workability and the like.

In the invention, the ingestion of a protein containing a phosphorylated amino acid such as CPP or the like to mammals makes it possible to enhance an immunity, increase a resistance to infectious diseases, remove growth inhibitory factors of mammals and promote growth.

Further, IgA and IgG are increased by administration of a protein containing a phosphorylated amino acid. Accordingly, infectious diseases can be prevented by adding this to appropriate food or food ingredients and causing humans to ingest the same continuously. Likewise, infectious diseases can be prevented in dogs, cats and the like by mixing pet food with an appropriate amount of this substance.

## Description

### Technical Field

The present invention relates to a composition for growth promotion of mammals, and a method for growth promotion of mammals using the same. More specifically, it relates to a composition for growth promotion of domestic animals comprising a protein containing a phosphorylated amino acid, and a method in which an immunity of mammals is enhanced by administering the composition to mammals to promote growth.

### Background Art

For satisfactorily growing mammals such as domestic animals and the like, it is important to prevent infectious diseases. Infectious diseases of domestic animals and the like are triggered by interrelation of various factors such as pathogens, hosts, environments and the like. In order to enhance growth of domestic animals and the like by preventing such infectious diseases, immunopotentiators have been so far added to a feed, and it has been known that various amino acids, colostrum preparations and the like are effective substances. Especially, a technique which has been widely practiced is addition of amino acids to a feed. However, this is problematic in both aspects of effects and costs, and the improvement has been in demand.

A milk casein phosphopeptide (hereinafter abbreviated as CPP) which is one type of proteins containing phospholyrated amino acids is a micropeptide having a high content of phosphoserine, which is obtained by reacting milk casein with an enzyme. The presence of CPP is identified also in fermentation food using milk as a raw material, and has a function to prevent insolubilization of minerals. Accordingly, it has attracted much interest as a substance for expediting absorption of calcium or the like.

Moreover, it has been known that CPP exhibits an immunological activity in a cell culture system, but so far unknown that when CPP is taken by being added to a feed etc. for domestic animals, antibody production is increased and a fattening effect is improved.

Further, adjuvants are to stimulate an immunological system to increase an immunoreaction against an antigen, and are mainly added to vaccines as an aid. As typical adjuvants, aluminum compounds, polynucleotides, cell ingredients of bacteria and the like are known. However, many of these are hardly used for humans. In particular, adjuvants are widely effective against antigenic substances. Therefore, when these are applied to vaccines for human bodies, they have problems such that there may be in danger of increasing antigenic stimulation of impurities contained in antigens or inducing a harmful allergic reaction so that great care must be taken of a purity of antigens used, and so forth. Thus, the development of adjuvants having a high safety has been in demand.

Not only are infectious diseases of domestic animals, such as pneumonia, bacterial diarrhea and the like quite disadvantageous in growth of the domestic animals, but also other healthy domestic animals might be infected therewith. Especially, infectious diseases attack infant animals because of their own weak immunity, and, in many cases, bring them to death.

Further, infectious diseases give a considerable influence of growth delay. In case of domestic animals, the economical loss is great. When pneumonia or bacterial diarrhea attacks domestic animals, treatment by administration of antibiotics is performed, which leads to increase in production costs, though. Accordingly, a method for immunopotentiation of domestic animals which is not problematic in safety, effects, workability, costs and the like has been in demand.

Under these circumstances, the present invention aims to establish a technology of enhancing an immunity of mammals such as domestic animals and the like, and thereby promoting growth.

### Disclosure of the Invention

The present inventors have assiduously conducted investigations to solve the foregoing problems, and have consequently found astonishingly that immunopotentiation is attained by orally administering CPP to domestic animals such as pigs and the like. This finding has led to the completion of the invention. Further, the use of CPP as an adjuvant has been also studied.

The invention recited in claim 1 is a composition for growth promotion of mammals characterized by comprising a protein containing a phospholyrated amino acid.

The invention recited in claim 2 is the composition recited in claim 1, wherein the protein containing the phosphorylated amino acid is a milk casein phosphopeptide.

The invention recited in claim 3 is a method for growth promotion of mammals characterized by administering the composition recited in claim 1 or 2 to mammals.

The invention recited in claim 4 is an immunoglobulin activation adjuvant characterized by comprising a protein containing a phosphorylated amino acid.

The invention recited in claim 5 is the adjuvant recited in claim 4, wherein the protein containing the phosphorylated amino acid is a milk casein phosphopeptide.

The invention recited in claim 6 is the adjuvant recited in claim 4, wherein the immunoglobulin is against β-lactoglobulin, Clostridium perfringens or Campylobacter coli.

### Brief Description of the Drawings

Fig. 1 is a graph showing a specific immunoglobulin antibody titer in intestinal contents (stool) of piglets treated with β-lactoglobulin.
Fig. 2 is a graph showing a total amount of IgA in intestinal contents (stool) of piglets.
Fig. 3 is a graph showing an amount of IgG in a serum of piglets.

### Best Mode for Carrying Out the Invention

The invention aims to cause mammals to ingest a protein containing a phosphorylated amino acid such as CPP or the like, whereby an immunity is enhanced, a resistance to infectious diseases is increased and growth inhibitory factors of mammals are removed to promote growth.

Types of the infectious diseases referred to in the invention are not limited, and include all diseases which are caused such that microorganisms as a pathogen invade the surface or the inside of mammals and are fixed and proliferated there.

Moreover, the effects of the invention brought forth by the protein containing the phosphorylated amino acid are, as will be later described, identified mainly in pigs. However, the mammals intended by the invention are not limited to pigs, and also include humans and other domestic animals such as cattle, horses, sheep and the like, having the same immunological system as pigs. Further, pet animals such as dogs, cats and the like are also included in the invention.

The protein containing the phospholyrated amino acid as used in the invention means a protein containing an amino acid having phosphoric acid bound thereto, and is widely present in vivo. Specific examples of this protein include phosvitin, casein, CPP and the like.

Of these, CPP can be, as described earlier, a micropeptide (milk casein phosphopeptide) having a high content of phosphoserine obtained by reacting milk casein with an enzyme. For example, trade names, "Meiji CPP-I", "Meiji CPP-II" and "Meiji CPP-III" (all made by Meiji Seika Kaisha, Ltd.) can be used.

A method for administering a protein such as CPP or the like to mammals is generally a method in which a feed or the like is mixed with a protein and is orally ingested. A method in which a protein is added to a drink such as water or the like and is ingested is also available.

A feed or the like to be mixed with a protein such as CPP or the like is not particularly limited, and a commercial product is available. An appropriate product may be selected depending on types of mammals, a degree of their growth and the like.

The amount of the protein containing the phosphorylated amino acid to be mixed with a feed or the like shall be determined in consideration of types of mammals or the like. In case of using, for example, trade name "Meiji CPP-I" (made by Meiji Seika Kaisha, Ltd.) as CPP, the amount is appropriately 0.1 to 1 % (by weight), preferably 0.5 % (by weight) based on a feed or the like.

Further, a period of administration of CPP to domestic animals and the like is not particularly limited. For expecting satisfactory effects, it is advisable that a CPP-containing feed is administered to domestic animals and the like continuously for a fixed period of time. For example, in case of piglets, the effects can be exhibited by oral administration for more than 3 weeks, preferably for 5 weeks.

The administration of the protein containing the phosphorylated amino acid increases IgA, and is effective for local immune. Moreover, when bacteria and the like are used as an antigen, IgG playing a main role in an antigen-antibody reaction is increased. In view of the foregoing, when this is administered to humans, infectious diseases can be prevented by adding it to food or food ingredients and continuously taking the same. Likewise, infectious diseases can be prevented in dogs, cats and the like by mixing pet food with an appropriate amount of this material.

The invention is illustrated more specifically below by referring to Examples to clarify the effects of the invention. However, the scope of the invention is not limited by these.

### Example 1

A CPP powder (trade name: "Meiji CPP-I" (CPP content: 15 %), made by Meiji Seika Kaisha, Ltd.) was added at a concentration of 0.5 % (by weight) to a feed for piglets shown in the following table according to age in week of piglets, and subjected to the following experiment.

**Table 1**

| Feed for growth of piglets | | |
|---|---|---|
| | for piglets 3 to 6 weeks old | for piglets 7 to 8 weeks old |
| Com | 73.0 % | 59.48 % |
| Soybean oil cake | 12.0 | 32.5 |
| Concentrated whey protein | 11.0 | 2.0 |
| Rice bran oil cake | 0.0 | 0.06 |
| Animal oil | 4.0 | 5.96 |

The following experiment was conducted using 16 head (all 3 weeks old) of hybrid piglets (LWD) of F1 of Landrace and Large White and Duroc as one group.

The feed shown in the foregoing table was freely ingested for 5 weeks. Further, as a control, a CPP-free feed to which 0.5 % (by weight) of ovoalbumin was added for adjusting the protein content was likewise ingested. Still further, in a β-lactoglobulin treatment group, β-lactoglobulin was intramuscularly injected in the form of an emulsion with a Freunt incomplete adjuvant three times at intervals of 2 weeks.

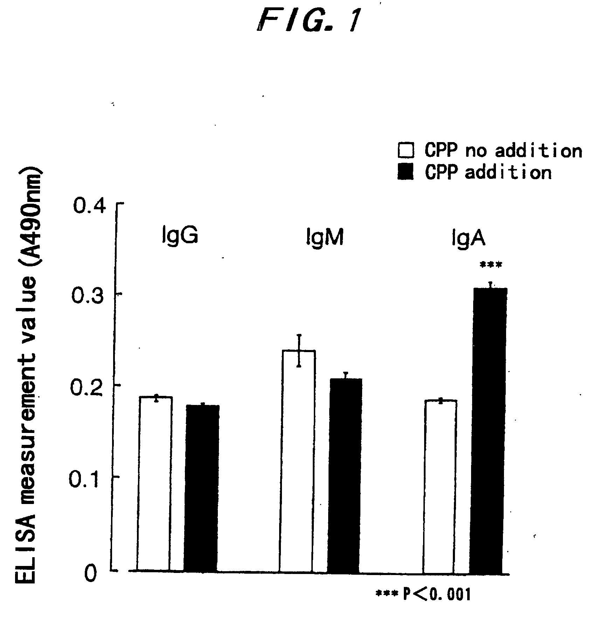

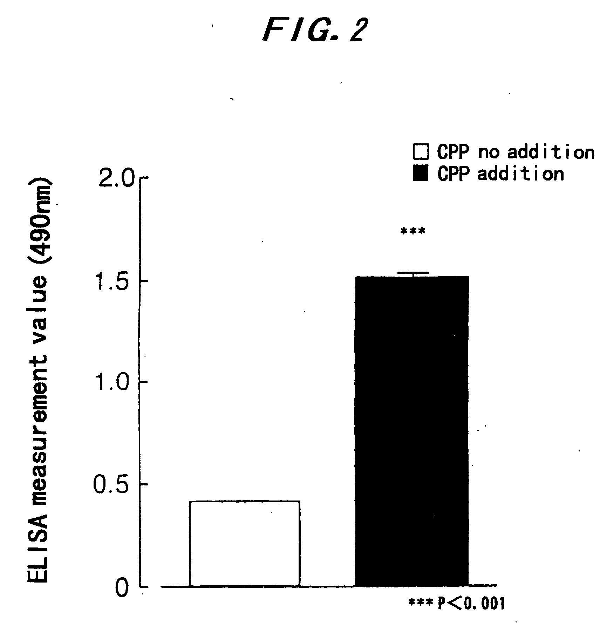

After breeding, the total amount of immunoglobulins in various classes and an amount of a specific antibody against β-lactoglobulin were measured by an enzyme-linked immunosorbent assay (ELISA). That is, the amounts of immunoglobulins (IgG, IgM and IgA) in the intestinal contents (stool) against β-lactoglobulin as measured by ELISA are shown in Fig. 1, and the total amount of IgA in the intestinal contents in Fig. 2.

As a result, almost no change was observed in the amounts of specific IgG and IgM in the intestinal contents against β-lactoglobulin by the administration of CPP, while the total amount of IgA was significantly increased. Further, the total amount of IgA in the intestinal contents was likewise increased.

By the way, IgA is known to play an important role in local immune. IgA is generated by a local mucosal lymphatic tissue, secreted to a mucosal surface as secretory IgA, and functions in a mucosal surface as a specific immune antibody against an antigen. It is said that secretory IgA is high in a bacterial or viral agglutination ability and also high in an affinity for mucus, and has further a resistance to digestive enzyme so that it is effective for inhibiting adhesion, invasion, colony formation and the like of microorganisms on a mucosal surface of intestines and the like (*buta byorigaku-seiri·shikkan·shiyo-* <3rd ed.>, published by K.K. Kindai Shuppan, pp. 78 - 91). It is indicated that immune is enhanced by the administration of CCP.

In addition, the weight of each individual was measured both at the start of the experiment of piglets (at the time of ablactation) and at the completion of the experiment, and the effect of growth promotion was estimated from the increase in weight. The results are shown in Table 2.

**Table 2**

| Results of measuring weight before and after experiment of piglets | | | | |
|---|---|---|---|---|
| | β-lactoglobulin treatment | Average weight in starting experiment (kg) | Average weight in completing experiment (kg) | Change in weight (kg) |
| CPP no addition | no | 7.46 | 12.68 | 5.22 |
| | yes | 7.50 | 13.04 | 5.54 |
| CPP addition | no | 7.48 | 15.86 | 8.38 |
| | yes | 7.62 | 14.94 | 7.32 |

As is clear from Table 2, the average weights in completing the experiment on piglets of the CPP no addition group were 12.68 kg and 13.04 kg, and the increased weights were 5.22 kg and 5.58 kg respectively. Meanwhile, the average weights in completing the experiment on piglets of the CPP addition group were 15.86 kg and 14.94 kg, and the increased weights were 8.38 kg and 7.32 kg respectively. Consequently, it was identified that the growth was promoted by the administration of CPP whether or not the β-lactoglobulin treatment was conducted.

### Example 2

In this Example, the following experiment was conducted for examining the immune activation effect of CPP, the safety of antigenic substances and the immunogenicity of antigenic substances.

First, as pigs to be tested, a total of 12 head of SPF piglets (average weight: 9.7 kg) which were at least 30 days old were used. As a feed for piglets, the same feed as in Example 1 was used. As a feed for a CPP administration group, a feed to which 0.5 % (by weight) of "Meiji CPP-I" (CPP content: 15 %, made by Meiji Seika Kaisha, Ltd.) was added as a CPP powder was used, while as a feed for a control group, a feed to which albumin (made by Sigma, hereinafter sometimes abbreviated as ALB) was likewise added to adjust a protein content was used. Incidentally, CPP or ALB was continuously added to the feed for 6 weeks from seven days before start-up of the test till termination of the test, and the feed was freely ingested.

Further, as antigenic substances, a Clostridium perfringens standard inactive strain (hereinafter sometimes abbreviated as a CL cell) and Campylobacter coli standard inactive strain (hereinafter sometimes abbreviated as a Cam cell) were used. These standard inactive strains were obtained by inactivating in a usual manner standard strains procured from National Institute of Animal Health, Ministry of Agriculture, Forestry and Fisheries, Japan. With respect to a method of administering these antigens, the administration was conducted by two routes, oral ingestion and subcutaneous injection for identifying the safety of antigenic substances. Test groups are shown in Table 3.

**Table 3**

| Test Groups | | |
|---|---|---|
| Group | Test groups | Head for test |
| CPP/CL+Cam group | CPP feed+antigen administration group (CL cell+Cam cell) | 2 head |
| CPP/CL group | CPP feed+antigen administration group (CL cell) | 5 head |
| ALB/CL group | ALB feed+antigen administration group (CL cell) | 2 head |
| ALB group | ALB feed control group | 3 head |

According to the foregoing test groups, in case of the CPP/CL + Cam group, or that is a CL cell and Cam cell administration group, a total cell amount of 8.5 mg (6 mg of CL cell + 2.5 mg of Cam cell) was administered in oral administration and a total cell amount of 6 mg (4 mg of CL cell + 2 mg of Cam cell) in subcutaneous injection at intervals of 1 week in such order that the oral administration was first conducted and the administration by injection was then conducted. Thus, the antigens were administered alternatively twice, and the antigen administration was conducted four times in total. Further, in case of the CPP/CL group and the ALB/CL group, or that is CL cell administration groups, a total cell amount of 6 mg was likewise administered in oral administration, and a total cell amount of 4 mg was likewise administered in subcutaneous administration. In oral administration, a feed mixed with cells suspended in a fixed amount of distilled water was used. In administration by subcutaneous injection, a Freunt incomplete adjuvant thereof was used. Incidentally, during a breeding period of 35 days, a feed and water were freely ingested, and weight measurement and blood sampling were periodically conducted.

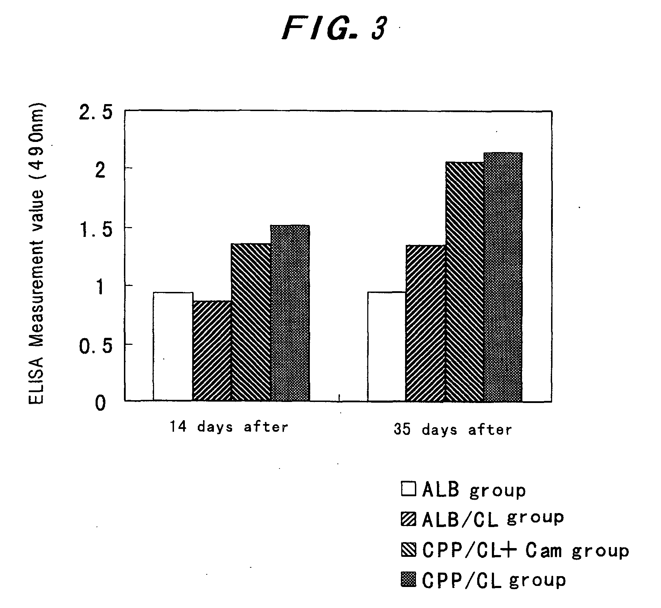

After breeding, amounts of specific antibodies against the CL cell and the Cam cell were measured by ELISA as in Example 1. IgG in a serum (diluted by a serum with 1,000 x magnification) measured by ELISA is shown in Fig. 3.

As a result, it was identified that IgG in the serum was increased in the CPP/CL + Cam group and CPP/CL group to which the CPP-containing feed was given in comparison with the ALB/CL group and the ALB group to which the ALB-containing feed was given. Further, as the period of administering antigens was prolonged, the amount of IgG was increased.

Moreover, the weight of each individual was measured in each group both at the start of the experiment of piglets and at the completion of the experiment of piglets. Consequently, in the CPP/CL + Cam group and the CPP/CL group with CPP administered, the weight was normally increased during the breeding period.

IgG is the most essential immunoglobulin in general animals, and occupies 80 % or more of immunoglobulins contained in a colostrum or a serum, playing a main role in the antigen-antibody reaction. Accordingly, IgG in a serum is increased by administering bacteria such as a CL cell, a Cam cell and the like as antigens, which proves that the immunity is enhanced by the administration of CPP.

### Industrial Applicability

The invention provides a composition for growth promotion of mammals and an adjuvant, comprising a protein containing a phosphorylated amino acid such as CPP or the like. By administering this composition to mammals, an immunity of mammals can be enhanced, and thereby growth can be promoted.

## Claims

1. A composition for growth promotion of mammals **characterized by** comprising a protein containing a phospholyrated amino acid.

2. The composition according to claim 1, wherein the protein containing the phosphorylated amino acid is a milk casein phosphopeptide.

3. A method for growth promotion of mammals **characterized by** administering the composition according to claim 1 or 2 to mammals.

4. An immunoglobulin activation adjuvant **characterized by** comprising a protein containing a phosphorylated amino acid.

5. The adjuvant according to claim 4, wherein the protein containing the phosphorylated amino acid is a milk casein phosphopeptide.

6. The adjuvant according to claim 4, wherein the immunoglobulin is an immunoglobulin against β-lactoglobulin, Clostridium perfringens or Campylobacter coli.
